# EUROPÄISCHE PATENTSCHRIFT

(11) **EP 0 699 206 B1**
(45) Veröffentlichungstag und Bekanntmachung des Hinweises auf die Patenterteilung: **13.08.1997**
(21) Anmeldenummer: 94917629.1
(22) Anmeldetag: 13.05.1994
(51) Int. Cl.: C07H 15/04, A61K 7/00, B01F 17/00

(54) **NICHTIONISCHE EMULGATOREN**
NON-IONIC EMULSIFIERS
EMULSIFIANTS NON IONIQUES

(30) Priorität: 21.05.1993 DE 4317089
(43) Veröffentlichungstag der Anmeldung: 06.03.1996
(73) Patentinhaber: Henkel Kommanditgesellschaft auf Aktien, 40191 Düsseldorf (DE)
(72) Erfinder: WEUTHEN, Manfred, D-42697 Solingen (DE); RIEGELS, Petra, D-42795 Leichlingen (DE); HARTEL, Irmgard, D-40589 Düsseldorf (DE)
(86) Internationale Anmeldenummer: EP9401552
(87) Internationale Veröffentlichungsnummer: WO9428006

(56) Entgegenhaltungen:
- EP-A- 0 165 721
- EP-A- 0 492 397
- WO-A-93/10133

## Beschreibung

### Gebiet der Erfindung

Die Erfindung betrifft nichtionische Emulgatoren, die man durch sauer katalysierte Acetalisierung von Zuckern mit überschüssigen Fettalkoholen bei Begrenzung des Umsatzes auf 60 bis 90 % der Theorie, Neutralisation und Filtration unter Rückvermischung erhält, ein Verfahren zu ihrer Herstellung sowie ihre Verwendung zur Herstellung oberflächenaktiver Mittel.

### Stand der Technik

Alkyloligoglykoside und insbesondere Alkyloligoglucoside stellen nichtionische Tenside dar, die infolge ihrer ausgezeichneten Detergenseigenschaften und ihrer hohen ökotoxikologischen Verträglichkeit zunehemend an Bedeutung gewinnen. Zu ihrer Herstellung wird von Zuckern oder Stärkeabbauprodukten ausgegangen, die üblicherweise in Gegenwart saurer Katalysatoren acetalisiert werden. Aus Gründen des Massenwirkungsgesetzes empfiehlt es sich, das Kondensationswasser kontinuierlich aus dem Reaktionsgleichgewicht zu entfernen und die eine Komponente, gewöhnlich den preiswerteren Fettalkohol, in ausreichendem Überschuß einzusetzen. Nach Abschluß der Reaktion wird der saure Katalysator beispielsweise mit Natriumhydroxid und/oder Magnesiumoxid neutralisiert und der überschüssige Fettalkohol gewöhnlich bis auf einen Restgehalt < 1 Gew.-% abdestilliert. Zur Herstellung hellfarbiger Produkte hat es sich in diesem Zusammenhang als erforderlich erwiesen, den Anteil nichtumgesetzten Zuckers auf 1, vorzugsweise 0,5 Gew.-% zu begrenzen oder anders formuliert, einen möglichst quantitativen Umsatz anzustreben.

Für den Kosmetiksektor haben sich als besonders hautverträgliche Emulgatoren neben den Alkyloligoglykosiden Tensidcompounds bewährt, die als zweite Komponente den korrespondierenden Fettalkohol enthalten. Die Herstellung solcher Mischungen ist vergleichsweise simpel und reduziert sich gewöhnlich darauf, den bei der Herstellung der Alky-oligoglykoside im Überschuß vorliegenden Fettalkohol im Produkt zu belassen.

So ist beispielsweise aus der **WO 92/06778** (Seppic) zur Herstellung von Emulsionen die Verwendung einer Mischung bekannt, die 60 bis 90 Gew.-% eines Fettalkohols mit 12 bis 22 Kohlenstoffatomen, 10 bis 90 Gew.-% eines Alkyloligoglykosides korrespondierender Kettenlänge sowie gegebenenfalls 0,5 bis 5 Gew.-% eines Glykosids enthält.

In der **DE-A1 40 33 928** (Henkel) werden ferner O/W-Emulsionen beansprucht, die 5 bis 30 Gew.-% eines wasserunlöslichen Ölkörpers, 2 bis 15 Gew.-% eines C8-C22-Alkyloligoglucosids, 2 bis 20 Gew.-% eines Fettsäurepartialglycerids sowie gegebenenfalls lineare gesättigte Fettalkohole enthalten.

Schon von der Herstellung der "reinen" Alkyloligoglykoside ist bekannt, daß diese nach Abtrennung des überschüssigen Fettalkohols starke Verfärbungen zeigen und mit Bleichmitteln behandelt werden müssen. Diese Problematik trifft - wenn auch nicht in gleichem Maße - für Compounds zu, die neben den Alkyl- und/oder Alkenyloligoglykosiden als zweite Komponente den überschüssigen Fettalkohol enthalten. Auch in diesem Fall ist eine Peroxidbleiche zur Herstellung hellfarbiger und für kosmetische Zwecke vermarktbarer Produkte in der Regel nicht zu umgehen.

Ein weiteres Problem, das ebenfalls von der Herstellung der Alkyloligoglykoside bekannt ist, stellen die langen Reaktionszeiten und die damit verbundene Kesselbelegung dar. Wie schon zuvor erwähnt, hat es sich als erforderlich erwiesen, den Gehalt an freier, nichtumgesetzter Glucose in der Reaktionsmischung zu minimieren, da diese sich während der Destillation zersetzen und zu farbgebenden Komponenten Anlaß geben kann; des weiteren kann es zur Bildung nennenswerter Anteile unerwünschter höherer Oligomere (Kondensationsgrad > 2) bis hin zur Polyglucose kommen.

Da eine destillative Abtrennung des Fettalkohols zur Herstellung der erfindungsgemäßen nichtionischen Emulgatoren jedoch ausdrücklich nicht vorgesehen ist, sondern freier Fettalkohol in der Mischung verbleiben soll, hat die Anmelderin versucht, den Umsatz zu begrenzen und die Reaktion nach einer definierten Zeit abzubrechen. Obschon auf diese Weise vergleichsweise hellfarbigere Produkte mit einem geringeren Gehalt an höheren Oligomeren erhalten werden, erfüllen diese doch nicht die hohen Qualitätsanforderungen, die an kosmetische Einsatzstoffe gestellt werden.

Die Aufgabe der Erfindung hat folglich darin bestanden, nichtionische Emulgatoren mit einem Gehalt an Alkyl- und/oder Alkenyloligoglykosiden und Fettalkoholen bereitzustellen, die sich durch hohe Farbqualität und einen niedrigen Anteil an Polyzuckern auszeichnen. Gleichzeitig sollten die Reaktionszeiten signifikant verkürzt werden.

### Beschreibung der Erfindung

Gegenstand der Erfindung sind nichtionische Emulgatoren, die man dadurch erhält, indem man
a) Zucker bzw. wäßrige Stärkeabbauprodukte und primäre Alkohole im molaren Verhältnis 1 2 bis 1 : 8, vorzugsweise 1 : 2,5 bis 1 : 4, in an sich bekannter Weise einer sauer katalysierten Acetalisierung unterwirft,
b) die Reaktion bei einem Umsatz im Bereich von 60 bis 90 % der Theorie abbricht,
c) das Reaktionsprodukt neutralisiert und unter Rückvermischung des Filtrates über ein Grobfilter heiß filtriert, bis das Filtrat klar erscheint.

Überraschenderweise wurde gefunden, daß sich hellfarbige Mischungen von Alkyl- und/oder Alkenyloligoglykosiden und den korrespondierenden Fettalkoholen mit einem geringen Anteil an höheren Oligomeren und Polyzuckern innerhalb verkürzter Reaktionszeiten erhalten lassen, wenn man die Reaktionszeit - und damit den Umsatz - wie angegeben begrenzt und die Produkte nach der Neutralisation unter Rückvermischung heiß filtriert. Die Erfindung schließt die Erkenntnis ein, daß sowohl Farbträger, als auch Oligomere an der nichtumgesetzten Glucose - dem Hauptbestandteil des Filterkuchens - adsorbiert werden, so daß nach mehrmaliger Rückvermischung ein blankes, farbloses Filtrat mit vermindertem Anteil an höheren Oligomeren bzw. Polyzuckern erhalten wird.

Ein weiterer Gegenstand der Erfindung betrifft ein Verfahren zur Herstellung von nichtionischen Emulgatoren, bei dem man
a) Zucker bzw. wäßrige Stärkeabbauprodukte und primäre Alkohole im molaren Verhältnis 1 : 2 bis 1 : 8, vorzugsweise 1 : 2,5 bis 1 : 4, in an sich bekannter Weise einer sauer katalysierten Acetalisierung unterwirft,
b) die Reaktion bei einem Umsatz im Bereich von 60 bis 90 % der Theorie abbricht,
c) das Reaktionsprodukt neutralisiert und unter Rückvermischung des Filtrates über ein Grobfilter heiß filtriert, bis das Filtrat klar erscheint.

### Einsatzstoffe

Als Einsatzstoffe für die Acetalisierung kommen Zucker mit 5 bis 6 Kohlenstoffatomen, vorzugsweise Glucose in Betracht.

Ferner geeignet sind auch wäßrige Stärkeabbauprodukte, wie beispielsweise Glucsoesirup mit einem Feststoffgehalt von 70 bis 90 Gew.-% und einem Monoglucosegehalt (DP1-Grad) von 90 bis 99 Gew.-%.

Als primäre Alkohole kommen Fettalkohole der Formel (I) in Frage,

R¹-OH (I)

in der R¹ für einen linearen oder verzweigten Alkyl- und/oder Alkenylrest mit 12 bis 22 Kohlenstoffatomen steht. Typische Beispiele sind Laurylalkohol, Myristylalkohol, Cetylalkohol, Palmoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol, sowie deren technische Gemische, wie sie beispielsweise bei der Hochdruckhydrierung von technischen Methylesterfraktionen oder Aldehyden aus der Roelen'schen Oxosynthese anfallen. Aus anwendungstechnischer Sicht bevorzugt sind Fettalkohole mit 16 bis 18 Kohlenstoffatomen. Im Hinblick auf das Emulgiervermögen sind Alkyloligoglucoside mit 16 Kohlenstoffatomen im Alkylrest und Fettalkohole mit 18 Kohlenstoffatomen besonders vorteilhaft. Im Sinne eines Compounds mit optimalen anwendungstechnischen Eigenschaften hat sich der Einsatz von Cetylstearylalkohol als Fettalkoholkomponente bewährt.

### Acetalisierung

Die Acetalisierung kann in an sich bekannter Weise erfolgen, indem man beispielsweise den Zucker vorlegt, gegebenenfalls entwässert, mit dem berechneten Überschuß an Fettalkohol versetzt, auf ca. 70 bis 80°C erhitzt und den sauren Katalysator, vorzugsweise gelöst in einer weiteren Portion Fettalkohol, kontinuierlich zudosiert. Die Acetalisierung wird üblicherweise bei Temperaturen im Bereich von 100 bis 110°C und unter vermindertem Druck (typischerweise 20 mbar) durchgeführt. Um die Reaktion in Richtung der gewünschten Produkte zuverlagern, ist es ferner sinnvoll, das Kondensationswasser kontinuierlich aus dem Gleichgewicht zu entfernen. Der Endpunkt der Reaktion, d. h. der angestrebte Umsatz, kann über das Verhältnis der Menge an abgeschiedenem Kondensationswasser zur theoretisch möglichen Menge berechnet werden. Im Sinne der Erfindung wird ein Umsetzungsgrad im Bereich von 60 bis 90 % der Theorie angestrebt; als optimal hat sich ein Bereich von 70 bis 80, vorzugsweise um 75 % der Theorie erwiesen. Es ist in diesem Zusammenhang selbstverständlich, daß ein für das Tensidcompound vorgegebener Gehalt an Alkyl- und/ oder Alkenyloligoglykosid sowohl über den Fettalkoholüberschuß, als auch über dem Umsatz angesteuert werden kann: bei großem Fettalkoholüberschuß wird ein definierter Gehalt an Acetalisierungsprodukt im Compound bei vergleichsweise höherem Umsatz erreicht, als umgekehrt. Die Abgleichung dieser beiden Parameter liegt jedoch innerhalb üblicher Optimierungsarbeiten und kann vom Fachmann durchgeführt werden, ohne hierzu erfinderisch tätig werden zu müssen.

Im Sinne der Erfindung lassen sich beispielsweise nichtionische Emulgatoren mit einem C_{16/18}-Alkyl- und/oder Alkenyloligoglykosidgehalt von ca. 22 Gew.-% bei Einsatz von Glucose und C_{16/18}-Fettalkohol im molaren Verhältnis 1 : 2,8 und einem Umsatz von 75 % der Theorie erhalten.

### Aufarbeitung

Nach Abbruch der Acetalisierung - durch Abkühlen des Reaktors und/oder Brechen des Vakuums - wird die saure Reaktionsmischung durch Zugabe einer Base, beispielsweise Natriumhydroxid und/oder Magnesiumoxid, neutralisiert und auf pH = 6 bis 7 eingestellt. Anschließend wird die nichtumgesetzte Glucose bei Temperaturen im Bereich von 70 bis 95, vorzugsweise 80 bis 90°C abfiltriert, wobei man sich üblicherweise eines Druckfilters konventioneller Bauart bedienen kann. Im Sinne des erfindungsgemäßen Verfahrens ist es erforderlich, das Filtrat im Kreis zu fahren und somit eine ausreichende Rückvermischung sicherzustellen, so daß sich ein Filterkuchen aufbauen kann, an dem Farbträger, höhere Oligomere und Polyzucker abdsorbiert werden. In einer besonderen Ausführungsform der Erfindung kann das Filtrat auch zwischen verschiedenen Filtereinheiten zirkuliert werden. Als Filtertypen kommen beispielsweise Tiefenfilter der Fa.Seitz in Betracht, wobei die Porenweite in einem Bereich von 10 bis 1000 µm liegen kann.

Der Endpunkt des Filtrationszyklusses ist erreicht, wenn das Filtrat klar ("blank") erscheint und eine weitere Verbesserung der Farbqualität nicht mehr beobachtet werden kann.

### Gewerbliche Anwendbarkeit

Die erfindungsgemäßen nichtionischen Emulgatoren können einen Gehalt von 25 bis 40 Gew.-% Alkyl- und/oder Alkenyloligoglykosiden und 75 bis 60 Gew.-% Fettalkohol aufweisen. Der durchschnittliche Polymerisationsgrad der Glucosidkomponente kann dabei 1 bis 10, vorzugsweise 1 bis 3 und insbesondere 1,1 bis 1,9 betragen.

Die nichtionischen Emulgatoren sind hellfarbig, ausgesprochen hautverträglich und bewirken die Vermischung von ansonsten nicht miteinander mischbarer Phasen. Gegenüber Produkten des Stands der Technik wirkt sich in diesem Zusammenhang der niedrige Gehalt an höheren Oligomeren und Polyzuckern besonders vorteilhaft aus.

Ein weiterer Gegenstand der Erfindung betrifft daher ihre Verwendung zur Herstellung von oberflächenaktiven Mitteln, beispielsweise Wasch-, Spül- und Reinigungsmitteln sowie insbesondere Mittel zur Haar- und Körperpflege, in denen sie in Mengen von 1 bis 50, vorzugsweise 3 bis 25 Gew.-% - bezogen auf die Mittel - enthalten sein können. Typische Beispiele sind in diesem Zusammenhang Haarshampoos, Badeöle, und Körperlotionen und -cremes.

Die folgenden Beispiele sollen den Gegenstand der Erfindung näher erläutern, ohne ihn darauf einzuschränken.

### Beispiele

### Beispiel 1:

1. **Katalysatorvorbereitung**. In einem 500-1-Rührkessel wurden 208 kg (812 mol) Cetylstearylalkohol (Lanette^{(R)} O, Henkel KGaA, Düsseldorf/FRG) auf 70°C erwärmt und unter Rühren mit 7,12 kg (36 mol) Sulfobernsteinsäure in Form einer 70 gew.-%igen wäßrigen Lösung versetzt. Die anfangs inhomogene Mischung wurde bei 70°C solange gerührt, bis sich die Sulfobernsteinsäure vollständig gelöst hatte (ca. 10 min).
2. **Acetalisierung.** In einem 10-m³-Reaktor mit Flüssigkeitszulauf, Rührwerk und Destillationsvorrichtung wurden 6184 kg (24.156 mol) Cetylstearylalkohol (geschuppte Ware) bei ca. 70°C aufgeschmolzen und unter Rühren mit 1600 kg (8888 mol) wasserfreier Glucose versetzt. Nach Anlegen eines Vakuums von 20 mbar wurde -die Temperatur auf 105°C gesteigert. Anschließend wurde die Katalysatorlösung aus (1) innerhalb von 30 min kontinuierlich zudosiert und das Reaktionswasser in eine Vorlage abdestilliert. Nach 5 bis 6 h und Erreichen eines Umsatzes von ca. 75 % (ermittelt über die theoretisch mögliche Menge an Kondensationswasser) wurde der Reaktor auf 85°C abgekühlt, das Vakuum aufgehoben und die Reaktion abgebrochen.
3. **Aufarbeitung.** Das saure Acetalisierungsprodukt wurde innerhalb von 2 h unter Zugabe von 0,64 kg Magnesiumoxid neutralisiert und auf pH = 6,5 eingestellt. Danach wurde der Ansatz bei einer Temperatur von ca. 90°C und einem Druck von 2 bar über ein Grobfilter (Porenweite : 10 µm] filtriert. Das Filtrat wurde dabei kontinuierlich in das Reaktionsgemisch zurückgepumpt, um eine Rückvermischung zu gewährleisten. Es wurden 368 kg Filterkuchen und 6824 kg Filtrat erhalten. Die Ergebnisse sind in Tab.1 zusammengefaßt.

### Vergleichsbeispiel V1:

Beispiel 1 wurde wiederholt. Das Acetalisierungsprodukt wurde neutralisiert, jedoch ohne Rückvermischung über einen Grobfilter filtriert. Die Ergebnisse sind in Tab.1 zusammengefaßt.

### Vergleichsbeispiel V2:

Beispiel 1 wurde wiederholt, die Reaktion jedoch erst nach 8 h und einem Umsatz von ca. 99 % abgebrochen. Das Acetalisierungsprodukt wurde neutralisiert und unter Rückvermischung über einen Grobfilter filtriert. Die Ergebnisse sind in Tab.1 zusammengefaßt.

### Vergleichsbeispiel V3:

Beispiel 1 wurde wiederholt, die Reaktion jedoch erst nach 8 h und einem Umsatz von ca. 99 % abgebrochen. Das Acetalisierungsprodukt wurde neutralisiert, jedoch ohne Rückvermischung über einen Grobfilter filtriert. Die Ergebnisse sind in Tab.1 zusammengefaßt.

**Tab.1:**

| Versuchsergebnisse | | | | | | | | |
|---|---|---|---|---|---|---|---|---|
| Bsp. | U % | t h | Zusammensetzung (Gew.-%) | | | | | Farbe Klett |
| | | | FA | DP1 | DP2 | DPX | Rest | |
| 1 | 75 | 5 | 67,7 | 14,9 | 3,8 | 1,4 | 12,2 | 15 |
| V1 | 75 | 5 | 64,8 | 14,1 | 3,9 | 2,1 | 15,1 | 75 |
| V2 | 99 | 8 | 66,3 | 12,8 | 3,6 | 5,3 | 12,0 | 190 |
| V3 | 99 | 8 | 66,3 | 10,9 | 3,8 | 6,8 | 13,1 | 210 |
| Legende: U = Umsatz t = Reaktionszeit FA = Fettalkohol DP1 = Monoglucosid DP2 = Diglucosid DPX = Oligoglucosid Farbe = Farbzahl, gemessen im Klettphotometer Proben 30 gew.-%ig in Toluol, 4-cm-Küvette | | | | | | | | |

## Patentansprüche

1. Nichtionische Emulgatoren, dadurch erhältlich, daß man
a) Zucker bzw. wäßrige Stärkeabbauprodukte und primäre Alkohole im molaren Verhältnis 1 : 2 bis 1 : 8 in an sich bekannter Weise einer sauer katalysierten Acetalisierung unterwirft,
b) die Reaktion bei einem Umsatz im Bereich von 60 bis 90 % der Theorie abbricht,
c) das Reaktionsprodukt neutralisiert und unter Rückvermischung des Filtrates über ein Grobfilter heiß filtriert, bis das Filtrat klar erscheint.

2. Verfahren zur Herstellung von nichtionischen Emulgatoren, bei dem man
a) Zucker bzw. wäßrige Stärkeabbauprodukte und primäre Alkohole im molaren Verhältnis 1 : 2 bis 1 : 8 in an sich bekannter Weise einer sauer katalysierten Acetalisierung unterwirft,
b) die Reaktion bei einem Umsatz im Bereich von 60 bis 90 % der Theorie abbricht,
c) das Reaktionsprodukt neutralisiert und unter Rückvermischung des Filtrates über ein Grobfilter reinigt, bis das Filtrat klar erscheint.

3. Verfahren nach Anspruch 2, **dadurch gekennzeichnet**, daß man als Zucker Glucose einsetzt.

4. Verfahren nach den Ansprüchen 2 und 3, **dadurch gekennzeichnet**, daß man primäre Alkohole der Formel (I) einsetzt,
R¹-OH (I)
in der R¹ für einen linearen oder verzweigten Alkyl- und/oder Alkenylrest mit 12 bis 22 Kohlenstoffatomen steht.

5. Verfahren nach den Ansprüchen 2 bis 4, **dadurch gekennzeichnet**, daß man die Filtration bei 70 bis 95°C durchführt.

6. Verwendung von nichtionischen Emulgatoren nach Anspruch 1 zur Herstellung von oberflächenaktiven Mitteln.

## Claims

1. Nonionic emulsifiers obtainable by
a) subjecting sugars or aqueous starch degradation products and primary alcohols to acid-catalyzed acetalization in known manner in a molar ratio of 1:2 to 1:8,
b) terminating the reaction at a conversion of 60 to 90% of the theoretical,
c) neutralizing the reaction product and hot-filtering the neutralizate through a coarse filter with back-mixing of the filtrate until the filtrate appears clear.

2. A process for the production of nonionic emulsifiers in which
a) sugars or aqueous starch degradation products and primary alcohols are subjected to acid-catalyzed acetalization in known manner in a molar ratio of 1:2 to 1:8,
b) the reaction is terminated at a conversion of 60 to 90% of the theoretical,
c) the reaction product is neutralized and the neutralizate is hot-filtered through a coarse filter with back-mixing of the filtrate until the filtrate appears clear.

3. A process as claimed in claim 2, characterized in that glucose is used as the sugar.

4. A process as claimed in claims 2 and 3, characterized in that primary alcohols corresponding to formula (I):
R¹OH (I)
in which R¹ is a linear or branched alkyl and/or alkenyl group containing 12 to 22 carbon atoms,
are used.

5. A process as claimed in claims 2 to 4, characterized in that the filtration is carried out at 70 to 95°C.

6. The use of the nonionic emulsifiers claimed in claim 1 for the production of surface-active formulations.

## Revendications

1. Agents émulsionnants non ioniques que l'on peut obtenir :
a) en soumettant un sucre ou des produits aqueux de dégradation de l'amidon et des alcools primaires dans un rapport molaire allant de 1:2 à 1:8, d'une manière connue en soi à une acétalisation catalysée par un acide,
b) en interrompant la réaction pour un taux de conversion dans la zone de 60 à 90 % de la théorie,
c) en neutralisant le produit de réaction et en filtrant à chaud, tout en remélangeant le filtrat, sur un filtre grossier, jusqu'à ce que le filtrat apparaisse limpide.

2. Procédé de fabrication d'agents émulsionnants non ioniques dans lequel :
a) on soumet un sucre ou des produits aqueux de dégradation de l'amidon et des alcools primaires dans un rapport molaire allant de 1:2 à 1:8, à une acétalisation catalysée par un acide, d'une manière connue en soi,
b) on interrompt la réaction pour un taux de conversion dans la zone de 60 à 90 % de la théorie,
c) on neutralise le produit de réaction et tout en remélangeant le filtrat, on purifie sur un filtre grossier jusqu'à ce que le filtrat apparaisse limpide.

3. Procédé selon la revendication 2,
caractérisé en ce qu'
on met en oeuvre comme sucre, le glucose.

4. Procédé selon les revendications 2 et 3,
caractérisé en ce qu'
on met en oeuvre des alcools primaires de formule (I),
R¹-OH (I)
dans laquelle R¹ représente un radical alkyle et/ou alcényle ayant de 2 à 22 atomes de carbone.

5. Procédé selon les revendications 2 à 4,
caractérisé en ce qu'
on effectue la filtration de 70 à 95°C.

6. Utilisation d'agents émulsionnants non-ioniques selon la revendication 1 en vue de la fabrication d'agents tensioactifs.
